# EUROPEAN PATENT SPECIFICATION

(11) **EP 3 319 576 B1**
(45) Date of publication and mention of the grant of the patent: **02.10.2019**
(21) Application number: 16739271.1
(22) Date of filing: 01.07.2016
(51) Int. Cl.: A61J 1/20

(54) **LIQUID DRUG TRANSFER DEVICES FOR SECURE TELESCOPIC SNAP FIT ON INJECTION VIALS**
FLÜSSIGARZNEIMITTELTRANSFERVORRICHTUNGEN FÜR SICHERE TELESKOPISCHE SCHNAPPVERBINDUNG AUF INJEKTIONSPHIOLEN
DISPOSITIFS DE TRANSFERT DE MÉDICAMENT LIQUIDE POUR UN ÉLÉMENT À ENCLIQUETER TÉLESCOPIQUE SÉCURISÉ SUR DES FLACONS D'INJECTION

(30) Priority: 16.07.2015 IL 24000515
(43) Date of publication of application: 16.05.2018
(73) Proprietor: West Pharma. Services IL, Ltd., Ra'anana 4366411 (IL)
(72) Inventor: DAVID, Uri, 7408721 Nes Ziona (IL); DENENBURG, Igor, 70700 Gedera (IL)
(74) Representative: South, Nicholas Geoffrey
(86) International application number: PCT/IL2016/050709
(87) International publication number: WO 2017/009822

(56) References cited:
- US-A1- 2002 127 150
- US-B2- 8 070 739

## Description

### Field of the Invention

The invention relates to liquid drug transfer devices for secure telescopic snap fit on injection vials.

### Background of the Invention

ISO 8362-1 Injection containers and accessories - Part 1: injection vials made of glass tubing standardizes vial sizes, vial dimensions and vial tolerances. ISO 8362-1 defines the following terms: Vial tube, vial crown and vial neck intermediate a vial tube and a vial crown. The vial tube has a closed end and an Outer Diameter (OD) d1. The vial crown has a crown opening and an Outer Diameter (OD) d2. The vial neck has an Outer Diameter (OD) d3 and an Inner Diameter (ID) d4. The vial crown has an uppermost vial crown rim encircling the crown opening and a lowermost vial crown rim towards the vial neck. The crown opening has the same inner diameter as the vial neck. The injection vial has a vial shoulder between the vial neck and the vial tube. The vial tube and the vial shoulder meet at an uppermost vial tube rim. The diameters have the relationship: d1>d2>d3>d4. Most vial crown outer diameters are available in several vial tube outer diameters, for example, a vial crown outer diameter d2 = 20 mm is available in three standard vial tube outer diameters d1 = 22 or 24 or 30 mm. Accordingly, injection vials are referred to by their vial crown outer diameters and not their vial tube outer diameters.

Liquid drug transfer devices include a vial adapter having a transverse vial adapter top wall, a hollow puncturing cannula for puncturing a vial stopper and a downward depending skirt. The downward depending skirt extends sufficiently downward to shield a puncturing cannula tip to prevent inadvertent user contact therewith. The downward depending skirt is shaped and dimensioned to snugly telescopically snap fit onto a particular vial crown outer diameter. Accordingly, vial adapters in a similar manner to injection vials are referred to in terms of a vial crown outer diameter for which they are intended to telescopic snap fit thereon. To ensure a vial adapter can be telescopically snap fit onto all the standard vial tube outer diameters of a particular vial crown outer diameter, its downward depending skirt is dimensioned to terminate above a vial shoulder. However, such downward depending skirts do not assist a user to align a vial adapter co-axial with an injection vial leading to common misalignment. Misalignment of a vial adapter with respect to an injection vial typically leads to the formation of a tear in a vial stopper as discussed in *inter alia* commonly owned US Patent No. 8,608,723 to Lev et al. entitled Fluid Transfer Devices with Sealing Arrangement.

Commonly owned US Patent No. 8,070,739 to Zinger et al. entitled Liquid Drug Transfer Devices for Failsafe Correct Snap Fitting onto Medicinal Vials discloses liquid drug transfer devices having a vial adapter designed to assist a user to align a vial adapter with an injection vial. The liquid drug transfer devices include a vial adapter having a downward depending skirt with at least two non-adjacent vial retention flex members for snap fitting over a vial crown for vial retention purposes and at least two non-adjacent vial guidance flex members longer than their counterpart vial retention flex members for guiding a vial adapter with respect to an injection vial prior to snap fitting the vial adapter thereon.

During the snap fit on an injection vial, the vial guidance flex members typically abut a vial shoulder and slide radial outwards on being outwardly radially flexed with respect to their vial adapter centerline at their junctures with their vial adapter top wall. The outward radial sliding of vial guidance flex members is dependent on the slope of a vial shoulder with a steeper slope facilitating outward radial sliding. The outward radial flexing of the vial guidance flex members at the junctures with their vial adapter top wall leads to a detachment reaction force opposing a manual attachment force for telescopic snap fitting a vial adapter on an injection vial. The greater the diameter of an injection tube the greater a detachment reaction force which at best leads to weakening a telescopic snap fit and at worst can lead to detachment.

Moreover, the telescopic snap fit of a vial adapter on an injection vial is not uniform for all the vial tube outer diameters for a particular vial crown outer diameter because the vial guidance flex members are outwardly radially flexed differently for different vial tube outer diameters. Also, larger vial tube outer diameters cause a vial adapter telescopic snap fitted onto an injection vial to present an awkward gripping surface for a user holding the vial adapter, for example, for attaching and detaching a syringe.

There is therefore a need to provide liquid drug transfer devices with vial adapters for providing a secure telescopic snap fit on a vial crown for all standard vial tube outer diameters for a particular vial crown outer diameter.

### Summary of the Invention

The present invention is defined in the appended claims and is directed towards liquid drug transfer devices including a vial adapter designed for secure telescopic snap fit on an injection vial. The liquid drug transfer devices have a similar construction as hitherto described US Patent No. 8,070,739 liquid drug transfer devices but their vial guidance flex members are additionally provisioned with a purpose design hinged zone distanced from a vial adapter top wall for intentional hinging thereat on telescopic snap fitting on an injection vial as opposed to flexing at a vial adapter top wall. The hinged zones preclude a vial adapter developing a significant detachment reaction force opposing a manual attachment force as may arise in the case of hitherto described US Patent No. 8,070,739 liquid drug transfer devices. Accordingly, the liquid drug transfer devices of the present invention can be readily used with injection vials of all vial tube outer diameters for a specific vial crown outer diameter. The liquid drug transfer devices of the present invention can be provisioned with a puncturing cannula longer than its vial retention flex members and vial guidance flex members longer than its puncturing cannula for shielding same but without detracting from a secure telescopic snap fit on an injection vial.

The vial guidance flex members preferably have their hinged zones disposed opposite the at least partially circumferentially extending inwardly protruding vial retention ribs of their counterpart vial retention flex members such that vial guidance flex members also snugly encircle a vial crown on telescopic snap fitting a vial adapter on an injection vial without snap fitting thereon. Vial retention flex members can double as vial guidance flex members by also being formed with hinged zones in a similar manner to vial guidance flex members on the condition their hinged zones are necessarily disposed distal to their at least partially circumferentially extending inwardly protruding vial retention ribs with respect to a vial adapter top wall. Accordingly, liquid drug transfer devices of the present invention can be provisioned with vial retention flex members only with at least two vial retention flex members doubling as vial guidance flex members with hinged zones.

### Brief Description of Drawings

In order to understand the invention and to see how it can be carried out in practice, preferred embodiments will now be described, by way of non-limiting examples only, with reference to the accompanying drawings in which similar parts are likewise numbered, and in which:
Fig. 1 shows ISO 8362-1 Section 4 Dimensions figure defining a vial tube outer diameter d1, a vial crown outer diameter d2, a vial neck outer diameter d3 and a vial neck inner diameter d4;
Fig. 2 is a perspective view of a conventional 20 mm female vial adapter in accordance with US Patent No. 8,070,739;
Fig. 3 is a longitudinal cross section of Figure 2's 20 mm female vial adapter along line A-A in Figure 2;
Fig. 4 is a longitudinal cross section along line A-A in Figure 2 showing Figure 2's 20 mm female vial adapter telescopic snap fit on a 20 mm injection vial having a vial tube outer diameter d1 = 22 mm;
Fig. 5 is a longitudinal cross section along line A-A in Figure 2 showing Figure 2's 20 mm female vial adapter telescopic snap fit on a 20 mm injection vial having a vial tube outer diameter d1 = 30 mm;
Fig. 6 is a perspective view of a 20 mm female vial adapter in accordance with a first embodiment of the present invention;
Fig. 7 is a longitudinal cross section of Figure 6's 20 mm female vial adapter along line B-B in Figure 6;
Fig. 8 is a longitudinal cross section showing Figure 6's 20 mm female vial adapter prior to being telescopic snap fit on a 20 mm injection vial having a vial tube outer diameter d1 = 22 mm;
Fig. 9 is a longitudinal cross section showing Figure 6's 20 mm female vial adapter telescopic snap fit on Figure 8's injection vial;
Fig. 10 is a longitudinal cross section showing Figure 6's 20 mm female vial adapter prior to being telescopic snap fit on a 20 mm injection vial having a vial tube outer diameter d1 = 30 mm;
Fig. 11 is a longitudinal cross section showing Figure 6's 20 mm female vial adapter telescopic snap fit on Figure 10's injection vial;
Fig. 12 is a perspective view of a 20 mm female vial adapter in accordance with a second embodiment of the present invention;
Fig. 13 is a longitudinal cross section of Figure 12's 20 mm female vial adapter along line C-C in Figure 12;
Fig. 14 is a perspective view of a 20 mm female vial adapter in accordance with a third embodiment of the present invention;
Fig. 15 is a longitudinal cross section of Figure 14's 20 mm female vial adapter along line D-D in Figure 14;
Fig. 16 is a perspective view of a 20 mm female vial adapter in accordance with a fourth embodiment of the present invention;
Fig. 17 is a top plan view of Figure 16's female vial adapter;
Fig. 18 is a longitudinal cross section of Figure 16's 20 mm female vial adapter along line E-E in Figure 16;
Fig. 19 is a perspective view of a vented 20 mm female vial adapter in accordance with a fifth embodiment of the present invention; and
Fig. 20 is a perspective view of a liquid drug transfer device in accordance with a sixth embodiment of the present invention.

### Detailed Description of Preferred Embodiments of the Invention

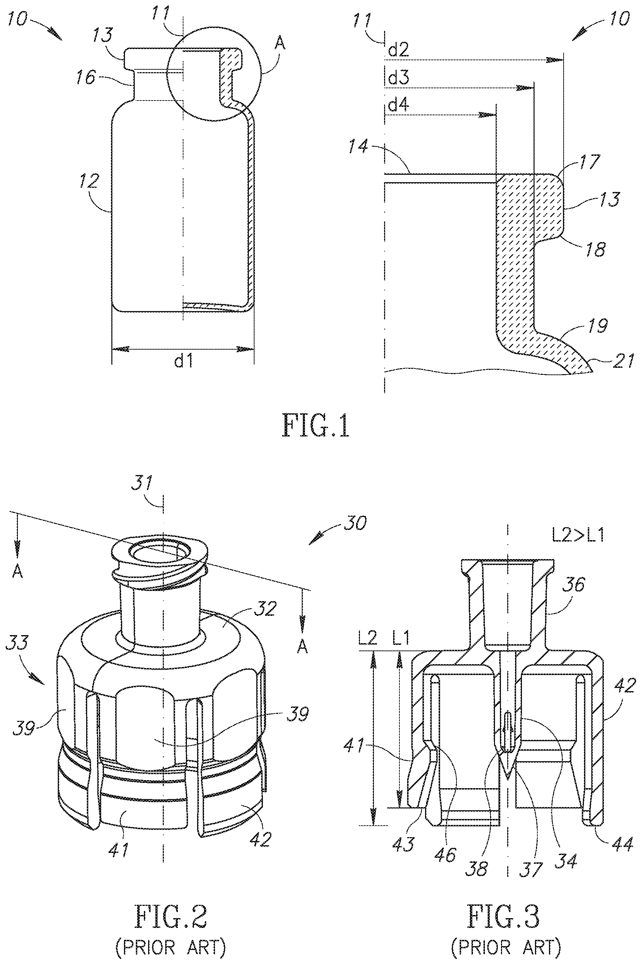

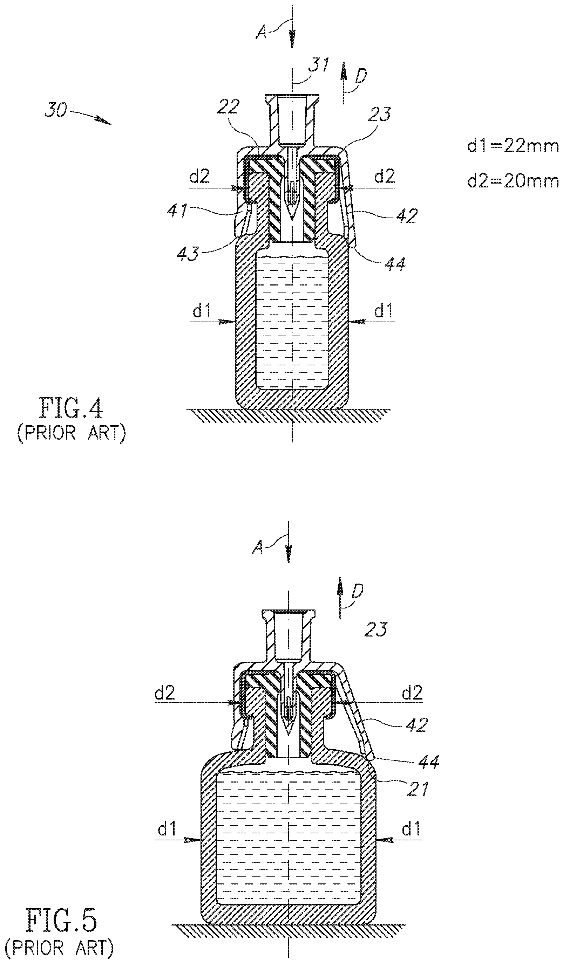

Figure 1 shows an injection vial 10 having a longitudinal injection vial centerline 11 and including a closed end vial tube 12, a tubular vial crown 13 having a crown opening 14 and a vial neck 16 intermediate the vial tube 12 and the vial crown 13. The vial crown 13 has an uppermost vial crown rim 17 encircling the crown opening 14 and a lowermost vial crown rim 18 towards the vial neck 16. The injection tube 10 includes a vial shoulder 19 intermediate the vial tube 12 and the vial neck 16. The vial tube 12 and the vial shoulder 19 meet at an uppermost vial tube rim 21. The vial tube 12 has an outer diameter d1. The vial crown 13 has an outer diameter d2. The vial neck 16 has an outer diameter d3 and an inner diameter d4. The crown opening 14 has the same inner diameter as the vial neck 16. The diameters have the relationship d1>d2>d3>d4. In the case of vial crown outer diameter d2 = 20 mm, the injection tube outer diameter d1 can be one of three standard diameters: d1 = 22 or 24 or 30 mm. Figures 4 and 5 show the injection vial 10 also includes a vial stopper 22 for stopping the crown opening 14 and an aluminum band 23 sealing the vial stopper 22.

Figures 2 and 3 show a conventional liquid drug transfer device 30 in accordance with hitherto mentioned US Patent No. 8,070,739 constituted by a female vial adapter for telescopic snap fit on a 20 mm injection vial. The female vial adapter 30 has a longitudinal vial adapter centerline 31 and includes a vial adapter top wall 32 transverse to the longitudinal vial adapter centerline 31 and a substantially cylindrical skirt 33 downwardly depending from the vial adapter top wall 32 for telescopically slidingly receiving a vial crown therein. The vial adapter top wall 32 includes a downward depending hollow puncturing cannula 34 for puncturing a vial stopper. The vial adapter top wall 32 includes a fluid transfer port 36 constituted by an upright female Luer connector opposite the puncturing cannula 34 and in flow communication therewith. The puncturing cannula 34 includes a puncturing cannula tip 37 with at least one flow aperture 38 for accessing a vial tube.

The skirt 33 includes six flex members 39 constituted by alternate vial retention flex members 41 and vial guidance flex members 42 equispaced around the longitudinal vial adapter centerline 31. The three vial retention flex members 41 and the three vial guidance flex members 42 occupy equal peripheral length around the longitudinal vial adapter centerline 31 in a top plan view of the female vial adapter 30. The vial retention flex members 41 have vial retention flex member tips 43 at a length L1 from the vial adapter top wall 32. The vial guidance flex members 42 have vial guidance flex member tips 44 at a length L2 from the vial adapter top wall 32 wherein L2>L1. The vial retention flex members 41 having partially circumferentially extending inwardly protruding vial retention ribs 46 for snap fitting over a vial crown for vial retention purposes.

Figures 4 and 5 show the 20 mm female vial adapter 30 correspondingly telescopic snap fit on 20 mm injection vials having a 22 mm vial tube outer diameter and a 30 mm vial tuber outer diameter. Figure 4 shows the vial guidance flex members 42 are slightly outwardly radially flexed at the vial adapter top wall 32 with respect to the vial adapter centerline 31 similar as shown in US Patent No. 8,070,739's Figure 3E. Figure 5 shows the vial guidance flex members 42 are more outwardly radially flexed at the vial adapter top wall 32 with respect to the vial adapter centerline 31 compared to Figure 4. The female vial adapter 30 presents a detachment reaction force denoted D opposing a manual attachment force denoted A. A greater detachment reaction force D is felt by a user in the case of Figure 5's 30 mm vial tube outer diameter than Figure 4's 20 mm vial tube outer diameter.

Figures 4 and 5 show the vial retention flex members 41 and vial guidance flex members 42 do not snugly embrace the vial crown 13 in a uniform manner. In Figure 4, since the vial tube outer diameter is only slightly greater than the vial crown outer diameter, the difference between the two types of flex members 41 and 42 is conceivably unnoticeable by a user holding the female vial adapter 30 during attachment and detachment of a syringe. In Figure 5, such outward radial flexing is highly noticeable by a user holding the female vial adapter 30 during attachment and detachment of a syringe thereby presenting an awkward gripping surface for a user holding the female vial adapter 30.

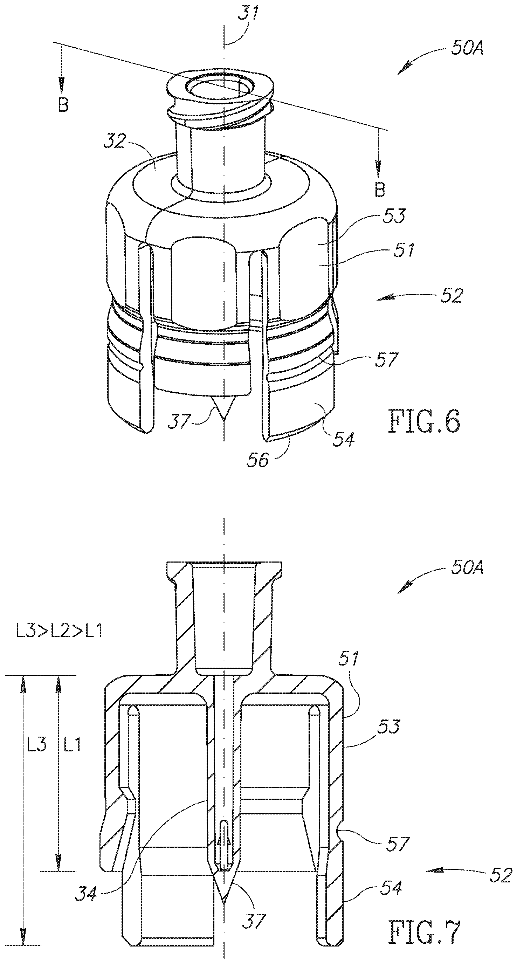

Figures 6 and 7 show a liquid drug transfer device 50A constituted by a female vial adapter for telescopic snap fit mounting on a 20 mm injection vial 10. The female vial adapter 50A has a similar construction and use as the female vial adapter 30 and therefore similar parts are likewise numbered. The female vial adapter 50A differs from the female vial adapter 30 insofar as the latter 50A has a puncturing cannula 34 longer than the vial retention flex members 41. Also the latter 50A includes vial guidance flex members 51 each having a hinged zone 52 dividing each vial guidance flex member 51 into an upper vial guidance flex member section 53 proximal the vial adapter top wall 32 and a lower vial guidance flex member section 54 distal the vial adapter top wall 32. The vial guidance flex members 51 terminate at vial guidance flex member tips 56 at a length L3 from the vial adapter top wall 32 where L3 > L2 > L1.

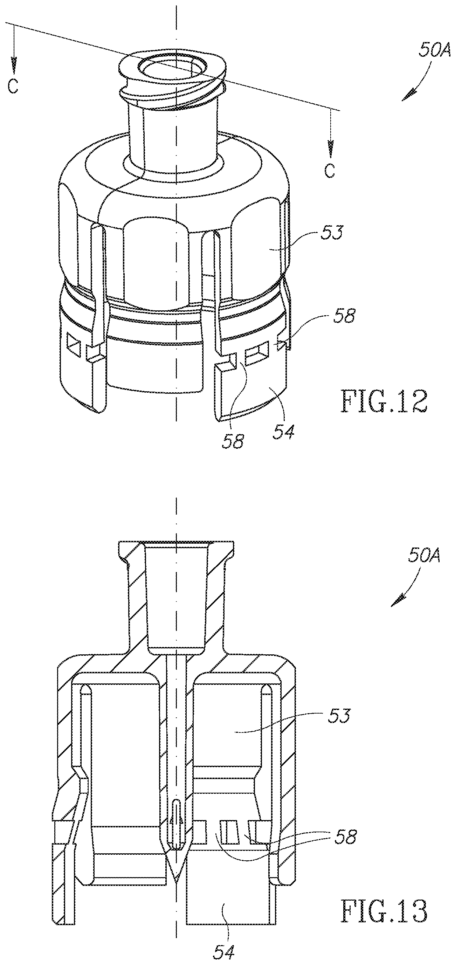

The vial retention flex members 41 having partially circumferentially extending inwardly protruding vial retention ribs 46 displaced from the vial adapter top wall 32. The hinged zones 52 are disposed substantially opposite the partially circumferentially extending inwardly protruding vial retention ribs 46. Accordingly, on telescopic snap fitting the female vial adapter 50A on a 20 mm injection vial 10, the hinged zones 52 are deployed between the lowermost vial crown rim 18 and the uppermost vial tube rim 21. The hinged zones 52 can be implemented, for example, by reducing material thickness, for example, by an external peripheral groove 57. Alternatively, the hinged zones 52 can be implemented by an upper vial guidance flex member 53 and a lower vial guidance flex member 54 being connected by two or more spaced apart hinges 58 as shown in Figures 12 and 13.

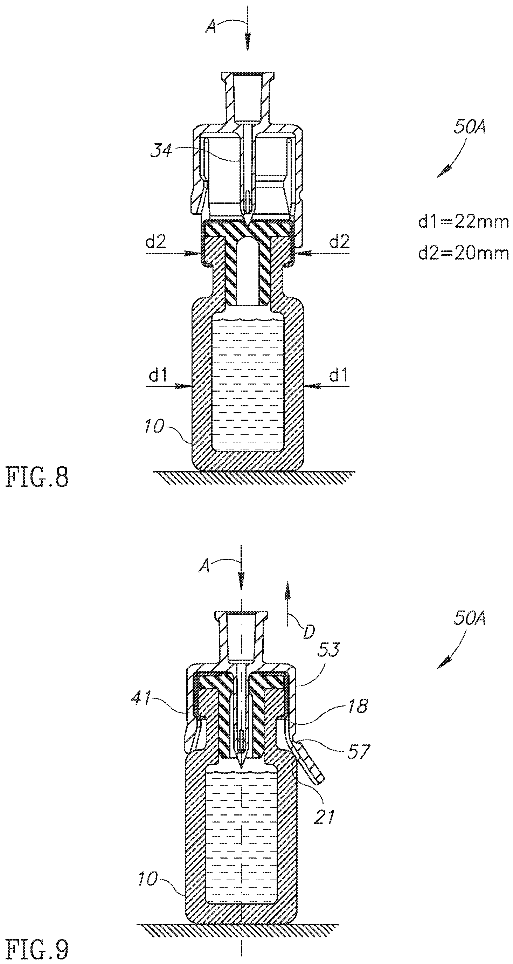

Figures 8 and 9 show the female vial adapter 50A before and after telescopic snap fitting on an injection vial 10 with a 20 mm vial crown outer diameter and a 22 mm vial tube outer diameter. Figure 8 shows the vial guidance flex members 51 assist centering of the female vial adapter 50A relative to the injection vial 10 as the female vial adapter 50A approaches the injection vial 10. On proceeding to depress the female vial adapter 50A towards the injection vial 10, the vial guidance flex members 51 initially slide down the aluminum band 23 until they contact the vial shoulder 19. The vial retention flex members 41 flex at their juncture with the vial adapter top wall 32 to snap fit over the vial crown 13 in a similar manner to the female vial adapter 30. Figure 9 shows the vial guidance flex members 51 are outwardly radially hinged at their respective hinged zones 52 relative to the longitudinal vial adapter centerline 31 as a result of the vial guidance flex member tips 56 contacting the vial shoulder 19 and sliding radial outward towards the uppermost vial tube rim 21 rather than being flexed at their juncture with the vial adapter top wall 32. A user may feel a slight detachment reaction force D as he applies an attachment force A. Figure 9 shows the upper vial guidance flex member sections 53 remain vertical similar to the vial retention flex members 41 unlike the vial guidance flex members 42 such that both the upper vial guidance flex member sections 53 and the vial retention flex members 41 snugly encircle the vial crown 13.

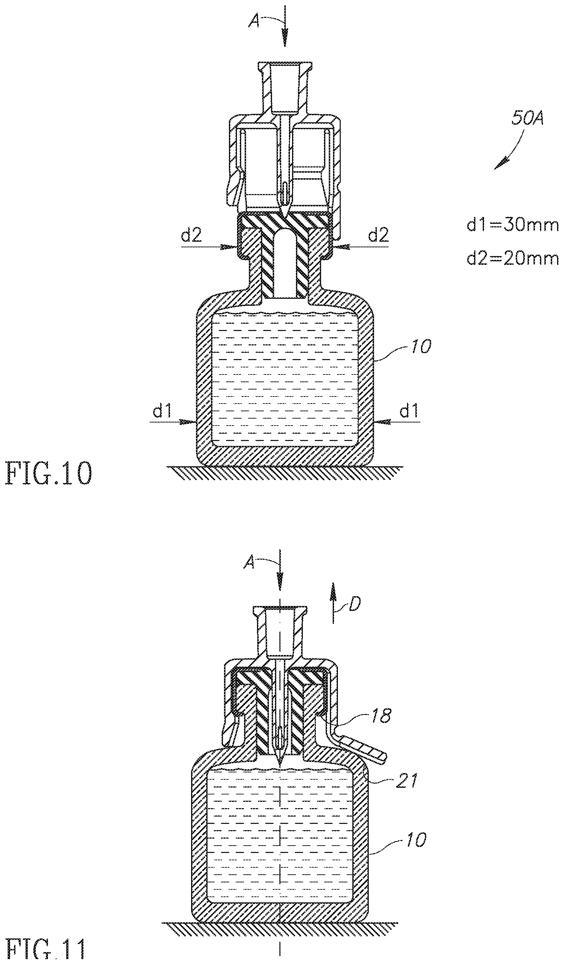

Figures 10 and 11 correspond to Figures 8 and 9 and differ therefrom insofar as they show the female vial adapter 50A before and after telescopic snap fitting on an injection vial 10 with the same 20 mm vial crown outer diameter but with a 30 mm vial tube outer diameter. Figure 11 shows the vial guidance flex members 51 are outwardly radially hinged at their respective hinged zones 52 relative to the longitudinal vial adapter centerline 31 as a result of the vial guidance flex member tips 56 contacting the vial shoulder 19 and sliding radial outwards towards the uppermost vial tube rim 21. A user may feel a slight detachment reaction force D as he applies an attachment force A considerably less than Figure 5's detachment reaction force D. Figure 11 shows the upper vial guidance flex member sections 53 remain vertical similar to the vial retention flex members 41 unlike the vial guidance flex members 42 such that both the upper vial guidance flex member sections 53 and the vial retention flex members 41 snugly encircle the vial crown 13. Thus, the female vial adapter 50A can be equally employed for telescopic snap fit on 20 mm injection vials of all its associated standard vial tube outer diameters 22 mm, 24 mm and 30 mm.

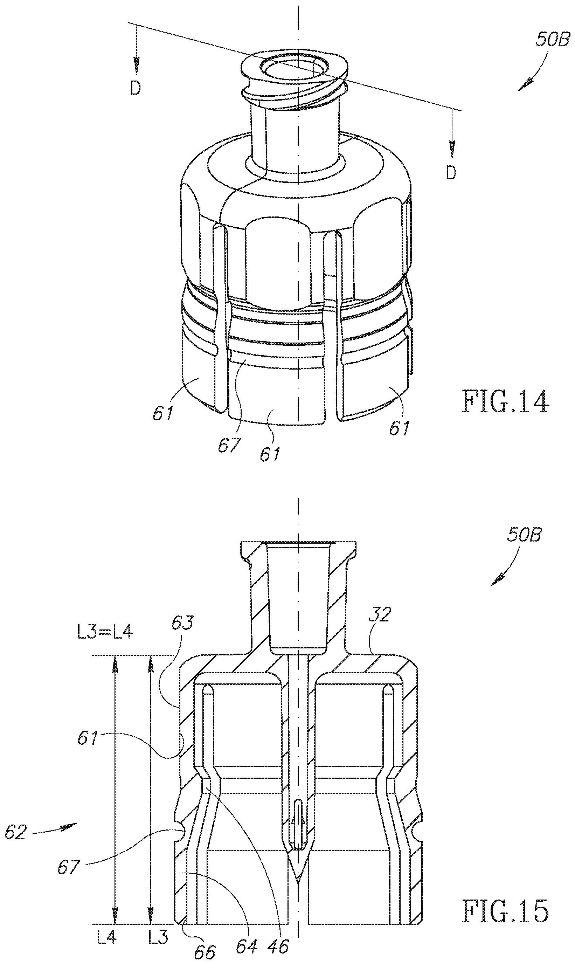

Figures 14 and 15 show a liquid drug transfer device 50B also constituted by a female vial adapter similar in construction and use as the female vial adapter 50A and therefore similar parts are likewise numbered. The latter 50B differs from the former 50A insofar as the latter 50B includes six vial retention flex members 61 and no vial guidance flex members. The six vial retention flex members 61 each include a hinged zone 62 similar to a hinged zone 52 thereby dividing each vial retention flex member 61 into an upper vial retention flex member section 63 proximal the vial adapter top wall 32 and a lower vial retention flex member section 64 distal the vial adapter top wall 32. The hinged zones 62 are necessarily disposed distal to the inwardly protruding vial retention ribs 46 with respect to the vial adapter top wall 32. The vial retention flex members 61 terminate at vial retention flex member tips 66 at a length L4 from the vial adapter top wall 32 where L4 = L3. The hinged zones 62 are constituted by an external peripheral groove 67.

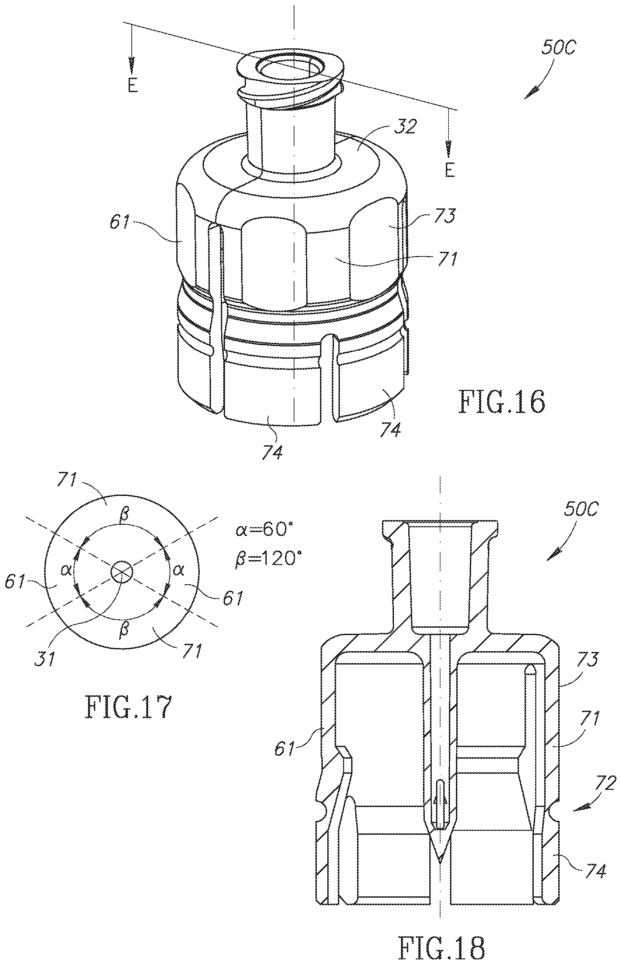

Figures 16 to 18 show a liquid drug transfer device 50C also constituted by a female vial adapter similar in construction and use as the female vial adapter 50B and therefore similar parts are likewise numbered. The female vial adapter 50C differs from the female vial adapter 50B insofar that it includes two diametric vial retention flex members 61 and two diametric vial guidance flex members 71. The two diametric vial guidance flex members 71 are designed to prevent any flexure at the vial adapter top wall 32 which may still occur in the case of the vial guidance flex members 51. Such prevention is achieved by the vial guidance flex members 71 subtend a section angle of at least 90° around the longitudinal vial adapter centerline 31 in Figure 17's top plan view. Typically, the two vial retention flex members 61 each subtend a sector angle α = 60° and the two vial guidance flex members 71 each subtend a sector angle β = 120°.

The vial guidance flex members 71 each include a hinged zone 72 dividing each vial guidance flex member 71 into an upper vial guidance flex member section 73 proximal the vial adapter top wall 32 and a pair of spaced apart lower vial guidance flex member sections 74 distal the vial adapter top wall 32. The vial guidance flex members 71 terminate at vial guidance flex member tips 76. The lower vial guidance flex member sections 74 outwardly radially hinge at their respective hinged zones 72 relative to the longitudinal vial adapter centerline 31 as a result of the vial guidance flex member tips 76 contacting a vial shoulder 19 and sliding radial outwards towards an uppermost vial tube rim 21 on telescopic snap fitting the female vial adapter 50C on an injection vial 10.

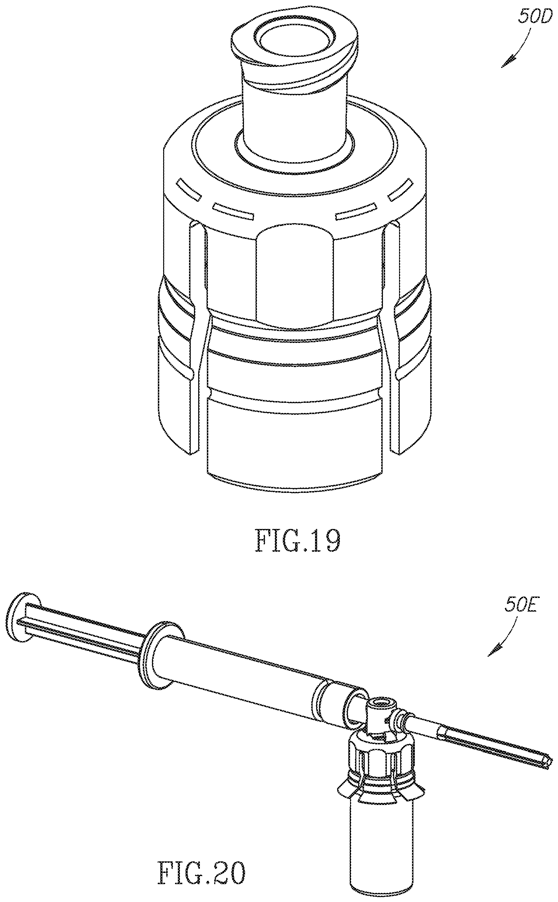

Figure 19 shows a liquid drug transfer device 50D constituted by a vented female vial adapter in accordance with the teachings of the present invention.

Figure 20 shows a liquid drug transfer device 50E constituted by a fluid control device disclosed in commonly owned US Patent No. 6,238,372 to Zinger et al. and including a vial adapter in accordance with the teaching of the present invention.

While particular embodiments of the present invention are illustrated and described, it would be obvious to those skilled in the art that various other changes and modifications can be made without departing from the scope of the invention.

## Claims

1. A liquid drug transfer device (50A; 50B; 50C; 50D; 50E) for secure telescopic snap fitting on an injection vial (10) having a longitudinal injection vial centerline (11) and including a closed end vial tube (12), a tubular vial crown (13) having a crown opening (14) stopped by a vial stopper (22), a vial neck (16) intermediate the vial tube and the vial crown, a vial shoulder (19) intermediate the vial tube and the vial neck, the vial crown having an uppermost vial crown rim (17) towards the crown opening and a lowermost vial crown rim (18) towards the vial neck, the vial tube and the vial shoulder meeting at an uppermost vial tube rim (21),
the vial tube having a vial tube outer diameter d1, the vial crown having a vial crown outer diameter d2, the vial neck having a vial neck outer diameter d3 and a vial neck inner diameter d4 wherein d1 >d2>d3>d4 and the vial tube outer diameter d1 being selected from a predetermined range of at least one vial tube outer diameter d1 for the vial crown outer diameter d2, the liquid drug transfer device comprising:
(a) a vial adapter having a longitudinal vial adapter centerline (31) and including a vial adapter top wall (32) transverse to said longitudinal vial adapter centerline, a substantially cylindrical skirt (33) downwardly depending from said vial adapter top wall for telescopically slidingly receiving the vial crown therein, and a hollow puncturing cannula (34) for puncturing the vial stopper, said puncturing cannula having a puncturing cannula tip (37) with at least one flow aperture (38) for accessing the vial tube,
said skirt including
i) at least two non-adjacent vial retention flex members (41; 61) having vial retention flex member tips (43; 66) distal to said vial adapter top wall, each said vial retention flex member having an at least partially circumferentially extending inwardly protruding vial retention rib (46) for snap fitting over the vial crown for vial retention purposes, and
ii) at least two non-adjacent vial guidance flex members (51; 71) being at least as long as said at least two non-adjacent vial retention flex members and having vial guidance flex member tips (56; 76) distal to said vial adapter top wall; and
(b) at least one fluid transfer port (36) in flow communication with said puncturing cannula,
**characterised by**
said at least two non-adjacent vial guidance flex members (51; 71) each having a hinged zone (52; 62; 72) located therealong distanced from said vial adapter top wall thereby dividing each said vial guidance flex member into an upper vial guidance flex member section (53; 73) proximal said vial adapter top wall and a lower vial guidance flex member section (54; 74) distal said vial adapter top wall,
said at least two non-adjacent vial guidance flex members being outwardly radially hinged at its respective hinged zone (52; 62; 72) relative to the longitudinal vial adapter centerline (31) on said vial guidance flex member tips (56; 76) contacting the vial shoulder (19) on telescopic snap fitting said vial adapter on the injection vial (10).

2. A liquid drug transfer device (50A; 50B; 50C; 50D; 50E) for secure telescopic snap fitting on an injection vial (10) having a longitudinal injection vial centerline (11) and including a closed end vial tube (12), a tubular vial crown (13) having a crown opening (14) stopped by a vial stopper (22), a vial neck (16) intermediate the vial tube and the vial crown, a vial shoulder (19) intermediate the vial tube and the vial neck, the vial crown having an uppermost vial crown rim (17) towards the crown opening and a lowermost vial crown rim (18) towards the vial neck, the vial tube and the vial shoulder meeting at an uppermost vial tube rim (21),
the vial tube having a vial tube outer diameter d1, the vial crown having a vial crown outer diameter d2, the vial neck having a vial neck outer diameter d3 and a vial neck inner diameter d4 wherein d1 >d2>d3>d4 and the vial tube outer diameter d1 being selected from a predetermined range of at least one vial tube outer diameter d1 for the vial crown outer diameter d2, the liquid drug transfer device comprising:
(a) a vial adapter having a longitudinal vial adapter centerline (31) and including a vial adapter top wall (32) transverse to said longitudinal vial adapter centerline, a substantially cylindrical skirt (33) downwardly depending from said vial adapter top wall for telescopically slidingly receiving the vial crown therein, and a hollow puncturing cannula (34) for puncturing the vial stopper, said puncturing cannula having a puncturing cannula tip (37) with at least one flow aperture (38) for accessing the vial tube,
said skirt including
at least two non-adjacent vial retention flex members (61) having vial retention flex member tips (66) distal to said vial adapter top wall, each said vial retention flex member having an at least partially circumferentially extending inwardly protruding vial retention rib (46) for snap fitting over the vial crown for vial retention purposes; and
(b) at least one fluid transfer port (36) in flow communication with said puncturing cannula,
**characterised by**
said at least two non-adjacent vial retention flex members (61) each having a hinged zone (62) located therealong distanced from said vial adapter top wall thereby dividing each said vial retention flex member into an upper vial retention flex member section (63) proximal said vial adapter top wall and a lower vial retention flex member section (64) distal said vial adapter top wall,
said at least two non-adjacent vial retention flex members being outwardly radially hinged at its respective hinged zone (62) relative to the longitudinal vial adapter centerline (31) on said vial retention flex member tips (66) contacting the vial shoulder (19) on telescopic snap fitting said vial adapter on the injection vial (10); and
each said hinged zone being further displaced therealong from said vial adapter top wall (32) than its at least partially circumferentially extending inwardly protruding vial retention rib (46) such that each said vial retention flex member is outwardly radially hinged at its respective hinged zone relative to the longitudinal vial adapter centerline (31) on said vial retention flex member tips (66) contacting the vial shoulder (19) on telescopic snap fitting said vial adapter on the injection vial (10).

3. The device according to claim 1 wherein said at least two non-adjacent vial guidance flex members (51; 71) each have said hinged zone displaced therealong at the same distance from said vial adapter top wall (32) as said at least partially circumferentially extending inwardly protruding vial retention ribs (46) such that, on telescopic snap fitting said vial adapter on the injection vial (10), said at least two upper vial guidance flex member sections (53; 73) and said at least two vial retention flex members (41; 61) snugly encircle the vial crown (13) in a uniform manner for each vial tube outer diameter d1 from the predetermined range of at least one vial tube outer diameter d1.

4. The device according to claim 3 wherein each said vial guidance flex member (71) subtends a sector angle of at least 90° around the longitudinal vial adapter centerline and includes a pair of spaced apart lower vial guidance flex member sections (74).

5. The device according to any one of claims 1 to 4 wherein a said hinged zone is constituted by an external peripheral groove (57; 67).

6. The device according to any one of claims 1 to 4 wherein a said hinged zone is constituted by two or more spaced apart hinges (58).

## Patentansprüche

1. Transfervorrichtung für flüssige Arzneimittel (50A; 50B; 50C; 50D; 50E) zum sicheren Befestigen des teleskopischen Schnappverschlusses an einer Injektionsflasche (10) mit einer längsgerichteten Injektionsflaschen-Mittellinie (11) und mit einer Ampulle (12) mit geschlossenem Ende, einer röhrenförmigen Ampullenkrone (13) mit einer Kronenöffnung (14), die durch einen Ampullenstopfen (22) verschlossen ist, einem Ampullenhals (16) zwischen dem Röhrchen und der Ampullenkrone, eine Ampullenschulter (19) zwischen dem Röhrchen und dem Ampullenhals, wobei die Ampullenkrone einen obersten Ampullenkronenrand (17) in Richtung der Kronenöffnung und einen untersten Ampullenkronenrand (18) in Richtung des Ampullenhalses aufweist, wobei das Ampullenrohr und die Ampullenschulter an einem obersten Rand des Ampullenröhrchens (21) aneinanderstoßen,
wobei das Ampullenröhrchen einen Ampullenaußendurchmesser d1 aufweist, wobei die Ampullenkrone einen Ampullenkronenaußendurchmesser d2 aufweist, wobei der Ampullenhals einen Ampullenhalsaußendurchmesser d3 und einen Ampullenhalsinnendurchmesser d4 aufweist, wobei d1>d2>d3>d4 und der Ampullenaußendurchmesser d1 aus einem vorbestimmten Bereich von mindestens einem Ampullenaußendurchmesser d1 für den Ampullenkronenaußendurchmesser d2 ausgewählt sind, die Transfervorrichtung für flüssige Arzneimittel umfassend:
(a) einen Ampullenadapter mit einer längsgerichteten Ampullenadapter-Mittellinie (31) und mit einer oberen Ampullenadapterwand (32) quer zur längsgerichteten Ampullenadapter-Mittellinie, einer im Wesentlichen zylindrischen Schürze (33) in Abwärtsrichtung in Abhängigkeit von der oberen Ampullenadapterwand zur teleskopisch gleitenden Aufnahme der Ampullenkrone darin und einer hohlen Punktionskanüle (34) zum Punktieren des Ampullenstopfens, wobei die Punktionskanüle eine Punktionskanülenspitze (37) mit mindestens einer Durchflussöffnung (38) zum Zugänglichmachen des Ampullenrohrs aufweist,
wobei die Schürze
i) mindestens zwei nicht angrenzende Ampullen-Rückhaltebiegeelemente (41; 61) mit Ampullen-Rückhaltebiegeelement-Spitzen (43; 66) distal zu der oberen Wand des Ampullenadapters aufweist, wobei jedes Ampullen-Rückhaltebiegeelement eine sich zumindest teilweise in Umfangsrichtung erstreckende, nach innen vorstehende Ampullenhalterippe (46) zum Einrasten über die Ampullenkrone zum Halten der Ampullen aufweist, und
ii) mindestens zwei nicht angrenzende Ampullen-Führungsbiegeelemente (51; 71), die mindestens so lang sind wie die mindestens zwei nicht angrenzenden Ampullen-Rückhaltebiegeelemente und Ampullen-Führungsbiegeelement-Spitzen (56; 76) distal zu der oberen Wand des Ampullenadapters aufweisen; und
(b) mindestens eine Flüssigkeitstransferöffnung (36) in Fließverbindung mit der Punktionskanüle,
**gekennzeichnet durch**
die mindestens zwei nicht angrenzenden Ampullen-Führungsbiegeelemente (51; 71), die jeweils einen klappbaren Bereich (52; 62; 72) aufweisen, der sich entlang der oberen Wand des Ampullenadapters befindet, wodurch jedes der Ampullen-Führungsbiegeelemente in einen oberen Ampullen-Führungsbiegeelementabschnitt (53; 73) proximal zur oberen Ampullenadapterwand und einen unteren Ampullen-Führungsbiegeelementabschnitt (54; 74) distal zur oberen Ampullenadapterwand unterteilt ist,
wobei die mindestens zwei nicht angrenzenden Ampullen-Führungsbiegeelemente an ihrem jeweiligen klappbaren Bereich (52; 62; 72) in Bezug auf die Mittellinie (31) des längsgerichteten Ampullenadapters an den Spitzen (56; 76) des Ampullen-Führungsbiegeelements nach außen radial angelenkt sind, die die Ampullenschulter (19) an der teleskopischen Schnappbefestigung des Ampullenadapters an der Injektionsflasche (10) berühren.

2. Transfervorrichtung für flüssige Arzneimittel (50A; 50B; 50C; 50D; 50E) zum sicheren Befestigen des teleskopischen Schnappverschlusses an einer Injektionsflasche (10) mit einer längsgerichteten Injektionsflaschen-Mittellinie (11) und mit einer Ampulle (12) mit geschlossenem Ende, einer röhrenförmigen Ampullenkrone (13) mit einer Kronenöffnung (14), die durch einen Ampullenstopfen (22) verschlossen ist, einem Ampullenhals (16) zwischen dem Röhrchen und der Ampullenkrone, eine Ampullenschulter (19) zwischen dem Röhrchen und dem Ampullenhals, wobei die Ampullenkrone einen obersten Ampullenkronenrand (17) in Richtung der Kronenöffnung und einen untersten Ampullenkronenrand (18) in Richtung des Ampullenhalses aufweist, wobei das Ampullenrohr und die Ampullenschulter an einem obersten Rand des Ampullenröhrchens (21) aneinanderstoßen,
wobei das Ampullenröhrchen einen Ampullenaußendurchmesser d1 aufweist, wobei die Ampullenkrone einen Ampullenkronenaußendurchmesser d2 aufweist, wobei der Ampullenhals einen Ampullenhalsaußendurchmesser d3 und einen Ampullenhalsinnendurchmesser d4 aufweist, wobei d1>d2>d3>d4 und der Ampullenaußendurchmesser d1 aus einem vorbestimmten Bereich von mindestens einem Ampullenaußendurchmesser d1 für den Ampullenkronenaußendurchmesser d2 ausgewählt sind, die Transfervorrichtung für flüssige Arzneimittel umfassend:
(a) einen Ampullenadapter mit einer längsgerichteten Ampullenadapter-Mittellinie (31) und mit einer oberen Ampullenadapterwand (32) quer zur längsgerichteten Ampullenadapter-Mittellinie, einer im Wesentlichen zylindrischen Schürze (33) in Abwärtsrichtung in Abhängigkeit von der oberen Ampullenadapterwand zur teleskopisch gleitenden Aufnahme der Ampullenkrone darin und einer hohlen Punktionskanüle (34) zum Punktieren des Ampullenstopfens, wobei die Punktionskanüle eine Punktionskanülenspitze (37) mit mindestens einer Durchflussöffnung (38) zum Zugänglichmachen des Ampullenrohrs aufweist,
wobei die Schürze
mindestens zwei nicht angrenzende Ampullen-Rückhaltebiegeelemente (61) mit Ampullen-Rückhaltebiegeelement-Spitzen (66) distal zu der oberen Wand des Ampullenadapters aufweist, wobei jedes Ampullen-Rückhaltebiegeelement eine sich zumindest teilweise in Umfangsrichtung erstreckende, nach innen vorstehende Ampullenhalterippe (46) zum Einrasten über die Ampullenkrone zum Halten der Ampullen aufweist, und
(b) mindestens eine Flüssigkeitstransferöffnung (36) in Fließverbindung mit der Punktionskanüle,
**gekennzeichnet durch**
die mindestens zwei nicht angrenzenden Ampullen-Rückhaltebiegeelemente (61), die jeweils einen klappbaren Bereich (62) aufweisen, der sich entlang der oberen Wand des Ampullenadapters befindet, wodurch jedes der Ampullen-Rückhaltebiegeelemente in einen oberen Ampullen-Rückhaltebiegeelementabschnitt (63) proximal zur oberen Ampullenadapterwand und einen unteren Ampullen-Rückhaltebiegeelementabschnitt (64) distal zur oberen Ampullenadapterwand unterteilt ist,
wobei die mindestens zwei nicht angrenzenden Ampullen-Rückhaltebiegeelemente an ihrem jeweiligen klappbaren Bereich (62) in Bezug auf die Mittellinie (31) des längsgerichteten Ampullenadapters an den Ampullen-Rückhaltebiegeelement-Spitzen (66) nach außen radial angelenkt sind, die die Ampullenschulter (19) an der teleskopischen Schnappbefestigung des Ampullenadapters an der Injektionsflasche (10) berühren; und
wobei jeder klappbare Bereich weiter von der oberen Ampullenadapterwand (32) verschoben ist als seine sich zumindest teilweise in Umfangsrichtung erstreckende, nach innen vorstehende Ampullenhalterippe (46), sodass jedes der Ampullen-Rückhaltebiegeelemente nach außen radial an seinem jeweiligen klappbaren Bereich in Bezug auf die längsgerichtete Ampullenadapter-Mittellinie (31) an den Ampullen-Rückhaltebiegeelement-Spitzen (66), die die Ampullenschulter (19) an der teleskopischen Schnappbefestigung des Ampullenadapters an der Injektionsflasche (10) berühren, radial angelenkt ist.

3. Vorrichtung nach Anspruch 1, wobei die mindestens zwei nicht angrenzenden Ampullen-Führungsbiegeelemente (51; 71) jeweils den klappbaren Bereich entlang desselben um den gleichen Abstand von der oberen Ampullenadapterwand (32) verschoben haben, wie die sich mindestens teilweise in Umfangsrichtung erstreckenden, nach innen vorstehenden Ampullenhalterippen (46), sodass an der teleskopischen Schnappbefestigung der Ampullenadapter an der Injektionsflasche (10) die mindestens zwei oberen Ampullen-Führungsbiegeelementabschnitte (53; 73) und die mindestens zwei Ampullen-Rückhaltebiegeelemente (41; 61) die Ampullenkrone (13) in gleichmäßiger Weise für jeden Ampullenaußendurchmesser d1 aus dem vorbestimmten Bereich von mindestens einem Ampullenaußendurchmesser d1 umschließen.

4. Vorrichtung nach Anspruch 3, wobei jedes der Ampullen-Führungsbiegeelemente (71) einen Sektorwinkel von mindestens 90° um die längsgerichtete Ampullenadapter-Mittellinie herum aufweist und ein Paar von beabstandeten unteren Ampullen-Führungsbiegeelementabschnitte (74) beinhaltet.

5. Vorrichtung nach einem der Ansprüche 1 bis 4, wobei ein klappbarer Bereich durch eine äußere Umfangsnut (57; 67) gebildet wird.

6. Vorrichtung nach einem der Ansprüche 1 bis 4, wobei ein klappbarer Bereich aus zwei oder mehreren beabstandeten Gelenken (58) gebildet ist.

## Revendications

1. Dispositif de transfert de médicament liquide (50A; 50B; 50C; 50D; 50E) pour fixer solidement un raccord télescopique monté sur un flacon d'injection (10) ayant une ligne centrale de flacon d'injection longitudinale (11) et comprenant un tube de flacon d'extrémité fermé (12), une couronne de flacon tubulaire (13) ayant une ouverture de couronne (14) bouchée par un bouchon de flacon (22), un goulot de flacon (16) intermédiaire du tube de flacon et de la couronne de flacon, un épaulement de flacon (19) intermédiaire du tube de flacon et du goulot de flacon, la couronne de flacon ayant un rebord de couronne de flacon supérieur (17) vers l'ouverture de la couronne et un rebord de couronne de flacon inférieur (18) vers le goulot de flacon, le tube de flacon et l'épaulement de flacon se rencontrant au niveau d'un rebord de tube de flacon supérieur (21),
le tube de flacon ayant un diamètre extérieur de tube de flacon d1, la couronne de flacon ayant un diamètre extérieur de couronne de flacon d2, le goulot du flacon ayant un diamètre extérieur de goulot de flacon d3 et un diamètre intérieur du goulot de flacon d4, dans lequel d1>d2>d3>d4 et le diamètre extérieur de tube de flacon d1 sélectionné à partir d'une plage prédéterminée d'au moins un diamètre extérieur de tube de flacon d1 pour le diamètre extérieur de la couronne de flacon d2, le dispositif de transfert de médicament liquide comprenant :
(a) un adaptateur de flacon ayant une ligne centrale de l'adaptateur de flacon longitudinal (31) et incluant une paroi supérieure d'adaptateur de flacon (32) transversale à ladite ligne centrale de l'adaptateur de flacon longitudinal, une jupe substantiellement cylindrique (33) orientée vers le bas selon ladite paroi supérieure de l'adaptateur de flacon, destinée à y recevoir de façon télescopique et coulissante la couronne de flacon, et une canule de ponction de creux (34) pour la perforation du bouchon de flacon, ladite canule de ponction ayant une pointe de canule de perforation (37) dotée d'au moins une ouverture d'écoulement (38) pour accéder au tube de flacon,
ladite jupe incluant
i) au moins deux éléments flexibles de rétention de flacons non adjacents (41 ; 61) ayant des pointes d'élément flexible de rétention de flacons (43 ; 66) distales à ladite paroi supérieure d'adaptateur de flacon, chacun desdits éléments flexibles de rétention de flacon ayant des nervures de rétention de flacon faisant saillie s'étendant vers l'intérieur au moins partiellement de manière circonférentielle (46) pour un ajustement serré sur la couronne du flacon à des fins de rétention de flacon, et
ii) au moins deux éléments flexibles de guidage de flacon non adjacents (51 ; 71) au moins aussi longtemps que lesdits au moins deux éléments flexibles de la rétention de flacons non adjacents et ayant des pointes d'éléments flexibles de guidage de flacons (56 ; 76) distales vers ladite paroi supérieure d'adaptateur de flacon ; et
(b) au moins un port de transfert de liquide (36) en communication fluidique avec ladite canule de perforation,
**caractérisé par**
lesdits au moins deux éléments flexibles de guidage des flacons non adjacents (51; 71) chacun ayant une zone à charnière (52; 62; 72) situé distancé le long de ladite paroi supérieure de l'adaptateur de flacon, divisant ainsi chacun dudit élément flexible de guidage de flacon en une section d'élément flexible de guidage de flacon supérieur (53; 73) proximal à ladite paroi supérieure de l'adaptateur de flacon et une section d'élément flexible de guidage inférieur (54; 74) distale de ladite paroi supérieure de l'adaptateur de flacon,
lesdits au moins deux éléments flexibles de guidage des flacons non adjacents, étant articulés radialement vers l'extérieur au niveau de sa zone à charnière respective (52 ; 62 ; 72) par rapport à la ligne centrale de l'adaptateur de flacon longitudinal (31) sur lesdites pointes d'éléments flexibles de guidage de flacon (56 ; 76) en contact avec l'épaulement du flacon (19) sur un raccord télescopique monté sur ledit adaptateur de flacon sur le flacon d'injection (10).

2. Dispositif de transfert de médicament liquide (50A; 50B; 50C; 50D; 50E) pour fixer solidement un raccord télescopique monté sur un flacon d'injection (10) ayant une ligne centrale de flacon d'injection longitudinale (11) et comprenant un tube de flacon d'extrémité fermé (12), une couronne de flacon tubulaire (13) ayant une ouverture de couronne (14) bouchée par un bouchon de flacon (22), un goulot de flacon (16) intermédiaire du tube de flacon et de la couronne de flacon, un épaulement de flacon (19) intermédiaire du tube de flacon et du goulot de flacon, la couronne de flacon ayant un rebord de couronne de flacon supérieur (17) vers l'ouverture de la couronne et un rebord de couronne de flacon inférieur (18) vers le goulot de flacon, le tube de flacon et l'épaulement de flacon se rencontrant au niveau d'un rebord de tube de flacon supérieur (21),
le tube de flacon ayant un diamètre extérieur de tube de flacon d1, la couronne de flacon ayant un diamètre extérieur de couronne de flacon d2, le goulot du flacon ayant un diamètre extérieur de goulot de flacon d3 et un diamètre intérieur du goulot de flacon d4, dans lequel d1>d2>d3>d4 et le diamètre extérieur de tube de flacon d1 sélectionné à partir d'une plage prédéterminée d'au moins un diamètre extérieur de tube de flacon d1 pour le diamètre extérieur de la couronne de flacon d2, le dispositif de transfert de médicament liquide comprenant :
(a) un adaptateur de flacon ayant une ligne centrale de l'adaptateur de flacon longitudinal (31) et incluant une paroi supérieure d'adaptateur de flacon (32) transversale à ladite ligne centrale de l'adaptateur de flacon longitudinal, une jupe substantiellement cylindrique (33) orientée vers le bas selon ladite paroi supérieure de l'adaptateur de flacon, destinée à y recevoir de façon télescopique et coulissante la couronne de flacon, et une canule de ponction de creux (34) pour la perforation du bouchon de flacon, ladite canule de ponction ayant une pointe de canule de perforation (37) dotée d'au moins une ouverture d'écoulement (38) pour accéder au tube de flacon,
ladite jupe incluant
au moins deux éléments flexibles de rétention de flacons non adjacents (61) ayant des pointes d'élément flexible de rétention de flacons (66) distales à ladite paroi supérieure d'adaptateur de flacon, chacun desdits éléments flexibles de rétention de flacon ayant des nervures de rétention de flacon faisant saillie s'étendant vers l'intérieur au moins partiellement de manière circonférentielle (46) pour un ajustement serré sur la couronne du flacon à des fins de rétention de flacon, et
(b) au moins un port de transfert de liquide (36) en communication fluidique avec ladite canule de perforation,
**caractérisé par**
lesdits au moins deux éléments flexibles de guidage des flacons non adjacents (61) chacun ayant une zone à charnière (62) situé distancé le long de ladite paroi supérieure de l'adaptateur de flacon, divisant ainsi chacun dudit élément flexible de guidage de flacon en une section d'élément flexible de guidage de flacon supérieur (63) proximal à ladite paroi supérieure de l'adaptateur de flacon et une section d'élément flexible de guidage inférieur (64) distale de ladite paroi supérieure de l'adaptateur de flacon,
lesdits au moins deux éléments flexibles de guidage des flacons non adjacents, étant articulés radialement vers l'extérieur au niveau de sa zone à charnière respective (62) par rapport à la ligne centrale de l'adaptateur de flacon longitudinal (31) sur lesdites pointes d'éléments flexibles de guidage de flacon (66) en contact avec l'épaulement du flacon (19) sur un raccord télescopique monté sur ledit adaptateur de flacon sur le flacon d'injection (10) ; et
chacune desdites zones à charnière étant davantage déplacée le long de ladite paroi supérieure de l'adaptateur de flacon (32) par rapport au moins partiellement à la nervure de rétention de flacon faisant saillie s'étendant vers l'intérieur au moins partiellement de manière circonférentielle (46) de sorte que chacun dudit élément flexible de rétention de flacon soit articulé radialement vers l'extérieur au niveau de sa zone à charnière respective par rapport à la ligne centrale de l'adaptateur de flacon longitudinale (31) sur lesdites pointes d'éléments flexibles de rétention de flacon (66) en contact avec l'épaulement de flacon (19) sur le raccord télescopique monté sur ledit adaptateur sur le flacon d'injection (10).

3. Dispositif selon la revendication 1, dans lequel lesdits au moins deux éléments flexibles de guidage de flacons non adjacents (51; 71) chacun ayant ladite zone à charnière déplacée le long de la même distance de ladite paroi supérieure de l'adaptateur de flacon (32) tel que lesdites nervures de rétention de flacon faisant saillie s'étendant vers l'intérieur au moins partiellement de manière circonférentielle (46) de sorte que, sur le raccord télescopique monté sur ledit adaptateur sur le flacon d'injection (10), lesdites au moins deux sections d'éléments flexibles de guidage de flacon supérieures (53; 73) et lesdites au moins deux éléments flexibles de rétention de flacons (41; 61) encerclant étroitement la couronne de flacon (13) de manière uniforme pour chaque diamètre extérieur de tube de flacon d1 d'une plage prédéterminée d'au moins un diamètre extérieur de tube de flacon d1.

4. Dispositif selon la revendication 3, dans lequel chacun dudit élément flexible de guidage de flacon (71) sous-tend un angle sectoriel d'au moins 90° autour de la ligne centrale de l'adaptateur de flacon longitudinal et comprend une paire de sections d'éléments flexibles de guidage de flacon inférieur espacées (74).

5. Dispositif selon l'une quelconque des revendications 1 à 4, dans lequel ladite zone à charnière est constituée d'une rainure périphérique externe (57 ; 67).

6. Dispositif selon l'une quelconque des revendications 1 à 4, dans lequel ladite zone à charnière est constituée de deux ou de plusieurs charnières séparées (58).
